# EUROPEAN PATENT SPECIFICATION

(11) **EP 1 259 153 B1**
(45) Date of publication and mention of the grant of the patent: **01.12.2004**
(21) Application number: 01908039.9
(22) Date of filing: 01.03.2001
(51) Int. Cl.: A61B 5/00, G01S 5/14, G01C 22/00, G01S 1/00

(54) **AN EXPERT SYSTEM FOR THE INTERACTIVE EXCHANGE OF INFORMATION BETWEEN A USER AND A DEDICATED INFORMATION SYSTEM**
EXPERTENSYSTEM ZUM AUSTAUSCH VON INFORMATIONEN ZWISCHEN EINEM BENUTZER UND EINEM ZUGEORDNETEM INFORMATIONSYSTEM
SYSTEME EXPERT DESTINE A UN ECHANGE INTERACTIF D'INFORMATION ENTRE UN UTILISATEUR ET UN SYSTEME D'INFORMATION DEDIE

(30) Priority: 01.03.2000 IT BO000105
(43) Date of publication of application: 27.11.2002
(60) Divisional application: 04011264.1
(73) Proprietor: TECHNOGYM S.p.A., 47035 Gambettola (Forli) (IT)
(72) Inventor: ALESSANDRI, Nerio, I-47020 Longiano (IT)
(74) Representative: Lanzoni, Luciano
(86) International application number: PCT/IB2001/000282
(87) International publication number: WO 2001/064098

(56) References cited:
- EP-A- 0 710 465
- WO-A-00/62867
- WO-A-90/08361
- DE-A- 19 731 986
- US-A- 5 339 821
- US-A- 5 527 239
- US-A- 5 810 747

## Description

### Technical Field

The present invention relates to the sector of physical exercise for sports, preventive and/or rehabilitative purposes and in particular it pertains to an expert system for the interactive and personalised exchange of information between a user and an information system dedicated to processing information concerning the achievement and the maintenance of the person's efficiency.

### Background Art

Exercise machines of the most modem and advanced design, both of the professional type and of the amateur type, are devised in such a way as to allow the user to program his/her physical activities in relation with his/her own personal characteristics and requirements.

For this purpose machines are known which, although conceived for a general employment able to meet the diversified needs of a plurality of users, can then be set on a case by case basis to accommodate each individual user and the execution of a specific, individualised work program.

Within the scope of a more pronounced customisation and of the most rigorous possible assessment of the requirement for exercise activity necessary to reach and maintain the best condition of efficiency and well-being, a system for the individualised operation of such machines, already known from patent applications BO99A000179 and BO99A000180 in the name of the same Applicant, takes into consideration the user as a system capable of exchanging energy with the outside world, not strictly limited to the time interval during which an exercise activity is conducted, but also correlated and quantified with the work activity performed and with the user's regimen and diet.

The system takes into consideration personal information, for instance relating to the person's actual physiological conditions, and manages them together with a series of other parameters such as those concerning his/her nutrition and those concerning the energy expenditure linked with the person's actual daily lifestyle.

This known technique has yielded fully satisfactory results from the standpoint of the specialisation and quantification of activities to suit each individual, but it is susceptible to be further improved, as it has the limitation of intervening on the individual only a *posteriori*, not being able to influence *a priori* his/her behaviours, rationalising and correcting certain habits which are necessary for obtaining the maximum benefit from physical exercise.

In EP-A-0710465 is disclosed a patient monitoring systems while WO-A-90/08361 depicts an aparatus for remotely controlling the exercise session of a patient.

### Disclosure of Invention

The aim of the present invention therefore is to provide a system which is able to interact with the user in an intelligent manner, assisting and guiding him/her with every sort of information connected and aimed at achieving the maximum possible physical well-being. In other words, the system aims to provide the user with specific and personalised information which range from individually tailored dietary advice, to the personal programming of the exercise machines, to the highlighting and correction of incorrect, counterproductive and/or dangerous behaviours, all this not being necessary limited solely to the time during which the exercise machines are actually used, but also in all other moments of the user's day.

In accordance with the invention, this aim is achieved by an expert system for the interactive exchange of information between a user and an information system, characterised in that it comprises a memory storage device in which is implemented said information system dedicated to the processing of information pertaining to the achievement and maintenance of the person's efficiency; a portable communication terminal able to exchange information between the user and the information system, said terminal being provided with at least a processing unit; input means and output means connected to the processing unit and operatively positioned between the latter and the user; and information exchange means positioned between the processing unit and the memory storage device.

If the terminal is constituted, for instance, by a palm-top computer or by a cell phone, palm-top or of such size as to be wearable, the user may query the information system at any moment of his/her day and receive therefrom advice and suggestions specific for him/herself, based on activities actually carried out and/or currently being carried out. Naturally, such activities are not necessary sporting in nature. Therefore, the terminal is a tool that allows to obtain the maximum benefit in physiological and mental terms.

To facilitate the capability of interactive communication between the user and the information system to the farthest extent possible, input and output means are provided which range from a conventional keyboard to voice synthesisers to allow the vocal and audio transmission of information.

The memory storage device and the terminal can operate locally, remotely, and be also supported by wired or wireless information exchange means.

The terminal can be provided with an interface suitable for connection with an exercise station, thereby allowing the operative system to configure the station each time in an optimal manner for the user, if the station is constituted by a machine for cardiovascular training; vice versa, in such a way as to provide an optimised work table if the machine is constituted by an exercise equipment item or a generic counterweight machine.

The expert system comprises means for supervising the user's activities with simultaneous assistance, i.e. with adaptive interaction, which assist the user as a tutor with a continuous interaction able to guide him/her, provide advice, encourage him/her, address him/her towards behaviours that are scientifically correct and/or statistically validated by experience, always fully respecting the user's individuality.

This simultaneous assistance is implemented with graphic representation studied according to the user's particularities.

The aforesaid feedback supervising means with simultaneous assistance allow to perform tutorial functions which can replace the presence of the gym instructor with the possibility for the user to perform the exercise activity in optimal conditions, also from the standpoint of safety, in a strictly private environment which can even be located inside his/her home.

### Description of the Drawings

The technical features of the invention, according to the aforesaid aims, can be clearly noted from the content of the claims set out below and its advantages shall become more readily apparent in the detailed description that follow, made with reference to the accompanying drawings, which show an embodiment provided purely by way of non limiting example, in which:
- Figure 1 is an overall diagram, in function blocks, of the system of the invention;
- Figures 2, 3, 4 are block diagrams showing, with a greater level of detail, some characteristic components of the invention of Figure 1.

### Description of the Illustrative Embodiment

With reference to the accompanying drawings, the reference number 1 globally indicates an expert system for the interactive exchange of information between a user 2 and an information system 3 dedicated to processing information relating to the achievement and maintenance of the person's efficiency.

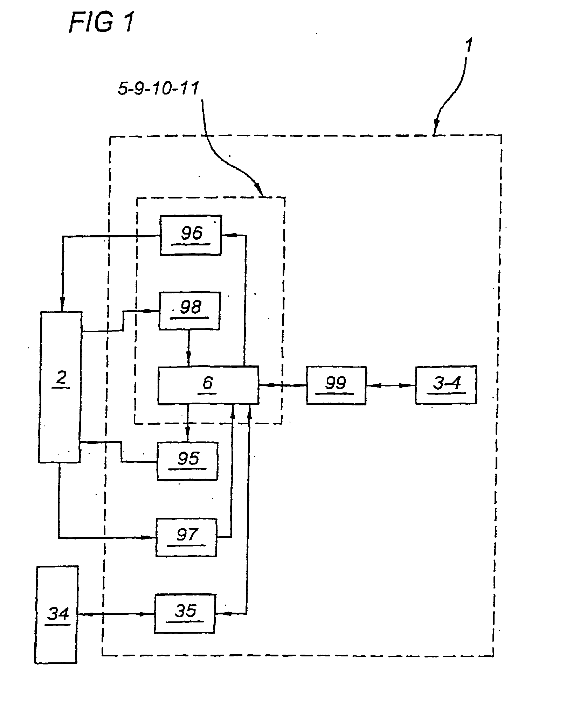

The system essentially comprises (see Figure 1): a memory storage device 4 in which the information system 3 is implemented; a terminal 5 for communicating with the user 2, provided with a processing unit 6; means for exchanging information 99 positioned between the terminal 5 and the memory storage device 4; and sensor means 97 to measure one or more of the state parameters of the user 2 and transmit them to the processing unit 6.

The memory storage device 4 and the terminal 5 are connected by the information exchange means 99 in different modes, which can be mutually combined also in different ways. The connection can be accomplished both locally and remotely; moreover, it can be obtained with a physical connection, in a network, via cable, or in wireless mode, for instance through the transmission of electromagnetic waves over the ether.

As to the embodiment of the memory storage device 4, different alternatives are possible.

The memory storage device 4 can be realised by a smart card from a personal computer; or by a server connected to a dedicated portal, through the Internet global network.

The communication terminal 5 between the user 2 and the information system 3 is provided, in turn (Figure 1), with at least a said microprocessor processing unit 6 and input means 98 and output means 96 connected to the processing unit 6 and operatively positioned between the latter and the user 2.

Constructively speaking, the communication terminal can be realised in numerous alternative ways: it can be of the stationary type and comprise for instance a personal computer 9; or it can be portable, embodied by a palm-top computer 10, by a cellular portable telephone 11, to be associated to a belt, so as to be wearable, for instance, at the user's wrist.

The communication terminal 5 can also be provided with an interface 35 to interact with at least an exercise station 34 dedicated to the user 2.

If the exercise station 34 is realised by a machine for cardiovascular training, the interface 35 is designed to manage also the training modes reproducible on the machine itself.

If, differently from this hypothesis; the exercise machine 34 instead comprises a counterweight machine, the terminal 5 provides the user 2 with instructions for executing a training program on said counterweight machine.

The interface 35 can be embodied in the most suitable forms, for the type of the exercise station 34, by means of an RS 232 card; a USB card; a Windows CE(™) standard compatible card or even an ADVANTECH(™) 5820/I card. Transmission protocols will be different in each case and may be identified for instance in the UMTS protocol for the transmission of video programs; in the WAP protocol usable for wireless communication, for instance with infrared beams, between computer and physical devices associated thereto. Said protocols may be employed, by way of non limiting indication, to allow the exchange of information for instance between communication terminal and information system or, or also, between input means, output means, processing unit and terminal.

The input means 98, associated to the terminal 5 and positioned between user 2 and processing unit 6, are also realised in a plurality of embodiments, which can mutually coexist also in various combinations.

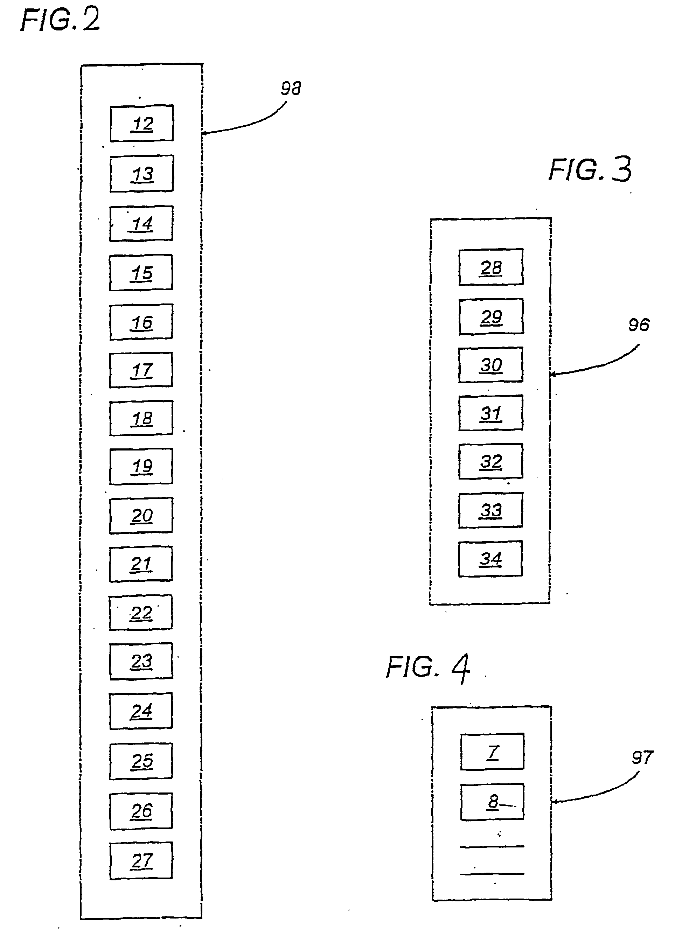

According to a first embodiment, said input means are constituted (see Figure 2) by a conventional keyboard 12 or, alternatively and/or additionally, they can be constituted: by a graphic tablet 13, possibly miniaturised; by a scanner 14, by an optical pen 15, by a joystick 16 (knob mounted on a base which can be moved with the fingers to control a cursor), by a track ball 17 (rotating ball which can be moved with the palm of the hand), by a mouse 18 (container provided with a ball whose movement allows to control a cursor), by a touch screen 19 (touch-sensitive device), by a magnetic reader 20, by an optical reader 21, by a video camera 22, by a voice synthesiser 23, by a CD-ROM reader 24, by a DVD reader 25, by a floppy disk reader 26 and by a modem 27. These input means 98 can be connected operatively with the terminal 5 which for this purpose is provided with suitable connection ports equipped with related cards.

The output means 96, associated to the terminal 5 and positioned between user 2 and processing unit 6 are embodied in their basic form by a monitor 28. However, they can also be realised in the form of LED or LCD display 29; or they can be embodied by a printer 30, by a plotter 31, by an audio synthesiser 32, by a master recording device 33 and by the same modem 27. All these means then interact with the terminal 5 by means of suitable ports and connection cards.

The system 1 further comprises sensor means 97 provided to measure one or more parameters relating to the physiological state of the user 2 and communicate them, in real time, to the information system 3 through the processing means 6.

According to the invention the sensor means 97, provided to measure a mechanical state of the user 2, in terms of instantaneous position parameters, provides for the use of a satellite position indicator 8. Knowledge of the instantaneous position of the user 2 allows the information system 3 to perform computations to determine the distances covered by the user during his/her daily ambulation or work activity, or during the execution of other activities such a walk or a run. This allows, taking into account the time and speeds of execution, assessments in terms of energy expenditure which will then be useful to determine a realistic requirement for exercise activity for the user, and consequently to enable the preparation of activity programs scientifically tailored for him/her.

The system comprises feedback means 95 for supervising the activity of the user 2 with simultaneous assistance of the user 2 him/herself. The user can be assisted and guided during the execution of his/her activity regardless of the physical presence of an instructor. This allows the great advantage of letting the user 2 perform his/her activity not necessarily on an exercise station 34 of a public gym, in the presence of an instructor, but also, possibly alone, in a personal home gym or even outdoors.

Said supervising means are designed to interact with the user 2 by means of an expressive graphic representation, preferably animated, which is conceived to provide differentiated simultaneous assistance, variable in relation with the specific characteristics of the user 2 or with the different age bands of the users 2 who interact with the information system 3. In other words, the animated graphics may vary in their expressive form depending on the type of user 2, in terms of their graphic aspect, their specific communication language, and their specific behaviour.

The supervising means are also designed to recognise the user 2, by means of self-learning consequent to his/her successive accesses to the information system 3; to store the interaction modes at each connection and also to perform comparisons of the performance of the user 2 with sample, reference, performance levels, which may for instance be constituted by a performance previously provided by the user 2 him/herself and stored by the information system 3; or by a performance which is being simultaneously provided by one or more other users 2: all this allowing the conduct of comparative tests or virtual competitions of the user 2 against him/herself or against other users 2 who exercise with him in a real or virtual training class, distributed in physically remote locations.

The idea at the basis of the present invention therefore is thus that of making available to the user 3 a knowledge base, residing in the information system 3, able to analyse the user's behavioural modes, to verify their correctness in relation to scientific principle and/or to statistically validated modes, intelligently guiding the user 2 towards the most suitable and effective behaviours for him/her.

The system further allows to implement a wide range of functionalities, able to be subdivided in broad terms, into specific functionalities of the exercise machine; into functionalities independent from the machine; and into functionalities correlated to the machine: irrespective of this classification, these functionalities are highly advantageously and strongly innovative in the sense that they integrate and expand some basic functionalities which, in principle, were already known, adding wholly new functionalities thereto.

Even for the specific functionalities of the exercise machine relating to controlling the machine and managing training modes - which in themselves are known from previous patent documents and embodiments by the Applicant - the degree of feedback effected on the exercise machine by the supervising means with simultaneous assistance to the user 2 is considerably amplified in its basic potentialities, and is also very effective and friendly by effect of the graphic/expressive capabilities of the system 1.

In regard to the functionalities that are independent from the exercise machine, in addition to the possibility of receiving virtual assistance, also in the form of simple personalised information, the system 1 allows the user 2 the opportunity, during the training session, to display the data for the session in real time in a browser window and to access basic Internet functionalities, thus being able to send and receive mail, or to communicate with other users by voice or video.

As to the functionalities correlated to the exercise machine of a generic station, a system according to the invention provides the capability of interfacing the machine with an Internet provider able to drive it.

Moreover, by realising a protocol able to allow to drive a machine remotely, the realisation is enabled of numerous other functionalities which foreshadow a real time link between the machine or the apparatus. It is possible to interact from a remote information system on the system for networking the machines in a gym, integrating with the local area network to conduct tests in real time; to propose new training programs; to re-propose previously conducted training sessions; to select a specific bitmap route; to select a training video; to run remote diagnostics on the machine and, or also, to replace the local control system with a centralised information system on a remote portal.

The invention thus conceived can be subject to numerous modifications and variations, without thereby departing from the scope of the inventive concept.

## Claims

1. An expert system for the interactive exchange of information, comprising:
an information system (3) dedicated to processing information relating to the achievement and maintenance of the person's efficiency;
a memory storage device (4) in which said information system (3) is implemented;
a portable communication terminal (5) between a user (2) and the information system (3), which is provided with at least a unit (6) for processing the information and input means (98) and output means (96) connected to the processing unit (6) and able to interact between it and the user (2);
means for exchanging information (99) positioned between the processing unit (6) and the memory storage device (4), to exchange information remotely;
sensor means (97) to measure at least a parameter of state of the user (2) and transmit it to the processing unit (6) and to the information system (3);
**characterised in that** the sensor means (97) measures at least one parameter of mechanical state of the user (2) and transmits it to the processing unit (6) and to the information system (3), to perform computation to determine the energy expenditure of the user (2) and to enable the preparation of activity programs scientifically tailored for the user (2); and **in that** the expert system comprises feedback means (95) for guiding the execution of the activity of the user (2) on the ground of said scientifically tailored activity programs.

2. An expert system as claimed in claim 1, **characterised in that** the sensor means (97) are devised to measure a parameter of instantaneous position of the user (2).

3. An expert system as claimed in claim 1, **characterised in that** the sensor means (97) comprise a satellite-type absolute position indicator (8).

4. An expert system as claimed in one of the previous claims 1 and 3-4, **characterised in that** the communication terminal (5) is stationary.

5. An expert system as claimed in claim 4, **characterised in that** the terminal (5) comprises a personal computer (9).

6. An expert system as claimed in claim 1, **characterised in that** said terminal (5) is a palm-top computer (10).

7. An expert system as claimed in claim 1, **characterised in that** the portable terminal (5) is a portable telephone (11).

8. An expert system as claimed in claim 7, **characterised in that** the portable telephone (11) is of the cellular type.

9. An expert system as claimed in one of the claims from 1 to 3 and 6 to 8, **characterised in that** said terminal (5) can be worn by the user (2).

10. An expert system as claimed in claim 9, **characterised in that** said terminal (5) is associated to a belt.

11. An expert system as claimed in claim 9, **characterised in that** said terminal (5) can be worn on the wrist of the user (2).

12. An expert system as claimed in one of the previous claims, **characterised in that** the input means (98) positioned between the user (2) and the processing unit (6) are embodied by a keyboard (12).

13. An expert system as claimed in claims 1-11, **characterised in that** the input means (98) positioned between the user (2) and the processing unit (6) are embodied by a graphic tablet (13).

14. An expert system as claimed in claims 1-11, **characterised in that** the input means (98) positioned between the user (2) and the processing unit (6) are embodied by a scanner (14).

15. An expert system as claimed in claims 1-11, **characterised in that** the input means (98) positioned between the user (2) and the processing unit (6) are embodied by an optical pen (15).

16. An expert system as claimed in claims 1-11, **characterised in that** the input means (98) positioned between the user (2) and the processing unit (6) are embodied by a joystick (16).

17. An expert system as claimed in claims 1-11, **characterised in that** the input means (98) positioned between the user (2) and the processing unit (6) are embodied by a track ball (17).

18. An expert system as claimed in claims 1-11, **characterised in that** the input means (98) positioned between the user (2) and the processing unit (6) are embodied by a mouse (18).

19. An expert system as claimed in claims 1-11, **characterised in that** the input means (98) positioned between the user (2) and the processing unit (6) are embodied by a touch screen (19).

20. An expert system as claimed in claims 1-11, **characterised in that** the input means (98) positioned between the user (2) and the processing unit (6) are embodied by a magnetic reader (20).

21. An expert system as claimed in claims 1-11, **characterised in that** the input means (98) positioned between the user (2) and the processing unit (6) are embodied by an optical reader (21).

22. An expert system as claimed in claims 1-11, **characterised in that** the input means (98) positioned between the user (2) and the processing unit (6) are embodied by a video camera (22).

23. An expert system as claimed in claims 1-11, **characterised in that** the input means (98) positioned between the user (2) and the processing unit (6) are embodied by a voice synthesiser (23).

24. An expert system as claimed in claims 1-11, **characterised in that** the input means (98) positioned between the user (2) and the processing unit (6) are embodied by a CD-ROM reader (24).

25. An expert system as claimed in claims 1-11, **characterised in that** the input means (98) positioned between the user (2) and the processing unit (6) are embodied by a DVD reader (25).

26. An expert system as claimed in claims 1-11, **characterised in that** the input means (98) positioned between the user (2) and the processing unit (6) are embodied by a floppy disk reader (26).

27. An expert system as claimed in claims 1-11, **characterised in that** the input means (98) positioned between the user (2) and the processing unit (6) are embodied by a modem (27).

28. An expert system as claimed in claims 1-27, **characterised in that** the output means (96) positioned between the user (2) and the processing unit (6) are embodied by a monitor (28).

29. An expert system as claimed in claims 1-27, **characterised in that** the output means (96) positioned between the user (2) and the processing unit (6) are embodied by a display (29).

30. An expert system as claimed in claims 1-27, **characterised in that** the output means (96) positioned between the user (2) and the processing unit (6) are embodied by a printer (30).

31. An expert system as claimed in claims 1-27, **characterised in that** the output means (96) positioned between the user (2) and the processing unit (6) are embodied by a plotter (31).

32. An expert system as claimed in claims 1-27, **characterised in that** the output means (96) positioned between the user (2) and the processing unit (6) are embodied by an audio synthesiser (32).

33. An expert system as claimed in claims 1-27, **characterised in that** the output means (96) positioned between the user (2) and the processing unit (6) are embodied by a master recording device (33).

34. An expert system as claimed in claims 1-27, **characterised in that** the output means (96) positioned between the user (2) and the processing unit (6) are embodied by a modem (27).

35. An expert system as claimed in one of the previous claims, **characterised in that** the means for exchanging information (99), between the memory storage device (4) and the terminal (5), comprise a physical connection via cable.

36. An expert system as claimed in claims 1-34, **characterised in that** the means for exchanging information (99) between the memory storage device (4) and the terminal (5) comprise a connection obtained in wireless mode.

37. An expert system as claimed in the claims from 35 to 36, **characterised in that** the memory storage device (4) is a server.

38. An expert system as claimed in claim 37, **characterised in that** the memory storage device (4) is an intranet server.

39. An expert system as claimed in one of the claims from 35 to 38, **characterised in that** the memory storage device (4) is an Internet server.

40. An expert system as claimed in one of the claims from 35 to 38, **characterised in that** the memory storage device (4) is a personal device.

41. An expert system as claimed in claim 40, **characterised in that** the memory storage device (4) is a smart card.

42. An expert system as claimed in one of the previous claims, **characterised in that** the communication terminal (5) is provided with an interface (35) to interact with at least an exercise station (34) dedicated to the user (2).

43. An expert system as claimed in claim 42, **characterised in that** the exercise station (34) comprises a cardiovascular training machine.

44. An expert system as claimed in claim 42, **characterised in that** the interface (35) is devised to manage at least the functionalities for controlling the cardiovascular training machine.

45. An expert system as claimed in claims 43 and 44, **characterised in that** the interface (35) is devised to manage the training modes reproducible on the cardiovascular training machine.

46. An expert system as claimed in claim 42, **characterised in that** the exercise station (34) comprises a counterweight machine, the terminal (5) being able to provide the user (2) with instructions for conducting a training program on said counterweight machine.

47. An expert system as claimed in at least one of the claims from 42 to 46, **characterised in that** said interface (35) is embodied by an RS 232 card.

48. An expert system as claimed in at least one of the claims from 42 to 46, **characterised in that** said interface (35) is embodied by a USB card.

49. An expert system as claimed in any of the previous claims, **characterised in that** it is able to employ a communication protocol (UMTS) able to transmit video programs.

50. An expert system as claimed in one of the previous claims **characterised in that** it is able to employ a communication protocol (WAP) able to transmit wireless signals.

## Patentansprüche

1. Expertensystem zum interaktiven Austausch von Informationen, enthaltend:
- ein Informationssystem (3), dazu bestimmt, eine sich auf das Erreichen und das Erhalten der Leistung einer Person beziehende Information zu verarbeiten;
- eine Speichervorrichtung (4), in welcher das genannte Informationssystem (3) umgesetzt wird;
- ein tragbares Terminal (5) zur Kommunikation zwischen einem Benutzer (2) und dem Informationssystem (3), welches mit wenigstens einer Einheit (6) zum Verarbeiten der Information sowie Eingangsmitteln (98) und Ausgangsmitteln (96) versehen ist, verbunden mit der Verarbeitungseinheit (6) und in der Lage, zwischen dieser und dem Benutzer (2) zusammenzuwirken;
- Mittel zum Austausch von Informationen (99), angeordnet zwischen der Verarbeitungseinheit (6) und der Speichervorrichtung (4), um die Informationen auf Entfernung auszutauschen;
- Fühlermittel (97) zum Erfassen von wenigstens einem Parameter des Zustands des Benutzers (2) und zum Übertragen desselben an die Verarbeitungseinheit (6) und an das Informationssystem (3);
**dadurch gekennzeichnet, dass** die Fühlermittel (97), wenigstens einen Parameter des mechanischen Zustands des Benutzers (2) aufnehmen und diesen an die Verarbeitungseinheit (6) und an das Informationssystem (3) übertragen, um eine Berechnung des Energieverbrauchs des Benutzers (2) durchzuführen und die Erarbeitung eines wissenschaftlich auf den Benutzer (2) zugeschnittenen Aktivitätenprogramms zu ermöglichen; und dadurch, dass das Expertensystem Rückkopplungsmittel (95) zum Leiten der Durchführung der Aktivität durch den Benutzer (2) aufgrund des genannten wissenschaftlich zugeschnittenen Aktivitätenprogramms enthält.

2. Expertensystem nach Patentanspruch 1, **dadurch gekennzeichnet, dass** die Fühlermittel (97) dazu vorgesehen sind, einen Parameter der augenblicklichen Position des Benutzers (2) zu erfassen.

3. Expertensystem nach Patentanspruch 1, **dadurch gekennzeichnet, dass** die Fühlermittel (97) einen satellitenartigen Anzeiger (8) der absoluten Position enthalten.

4. Expertensystem nach einem der vorstehenden Patentansprüche 1 und von 3-4, **dadurch gekennzeichnet, dass** das Kommunikationsterminal (5) stationär ist.

5. Expertensystem nach Patentanspruch 4, **dadurch gekennzeichnet, dass** das Terminal (5) einen Personalcomputer (9) enthält.

6. Expertensystem nach Patentanspruch 1, **dadurch gekennzeichnet, dass** das genannte Terminal (5) ein Palmtop (10) ist.

7. Expertensystem nach Patentanspruch 1, **dadurch gekennzeichnet, dass** das tragbare Terminal (5) ein tragbares Telefon (11) ist.

8. Expertensystem nach Patentanspruch 7, **dadurch gekennzeichnet, dass** das tragbare Telefon (11) ein Mobiltelefon ist.

9. Expertensystem nach einem der Patentansprüche von 1 bis 3 und von 6 bis 8, **dadurch gekennzeichnet, dass** das genannte Terminal (5) von dem Benutzer (2) getragen werden kann.

10. Expertensystem nach Patentanspruch 9, **dadurch gekennzeichnet, dass** das genannte Terminal (5) an einem Gürtel befestigt wird.

11. Expertensystem nach Patentanspruch 9, **dadurch gekennzeichnet, dass** das genannte Terminal (5) am Handgelenk des Benutzers (2) getragen werden kann.

12. Expertensystem nach einem der vorstehenden Patentansprüche, **dadurch gekennzeichnet, dass** die zwischen dem Benutzer (2) und der Verarbeitungseinheit (6) angeordneten Eingangsmittel (98) durch eine Tastatur (12) verkörpert sind.

13. Expertensystem nach den Patentansprüchen 1-11, **dadurch gekennzeichnet, dass** die zwischen dem Benutzer (2) und der Verarbeitungseinheit (6) angeordneten Eingangsmittel (98) durch eine graphische Tafel (13) verkörpert sind.

14. Expertensystem nach den Patentansprüchen 1-11, **dadurch gekennzeichnet, dass** die zwischen dem Benutzer (2) und der Verarbeitungseinheit (6) angeordneten Eingangsmittel (98) durch einen Scanner (14) verkörpert sind.

15. Expertensystem nach den Patentansprüchen 1-11, **dadurch gekennzeichnet, dass** die zwischen dem Benutzer (2) und der Verarbeitungseinheit (6) angeordneten Eingangsmittel (98) durch einen optischen Stift (15) verkörpert sind.

16. Expertensystem nach den Patentansprüchen 1-11, **dadurch gekennzeichnet, dass** die zwischen dem Benutzer (2) und der Verarbeitungseinheit (6) angeordneten Eingangsmittel (98) durch einen Joystick (16) verkörpert sind.

17. Expertensystem nach den Patentansprüchen 1-11, **dadurch gekennzeichnet, dass** die zwischen dem Benutzer (2) und der Verarbeitungseinheit (6) angeordneten Eingangsmittel (98) durch ein Trackball (17) verkörpert sind.

18. Expertensystem nach den Patentansprüchen 1-11, **dadurch gekennzeichnet, dass** die zwischen dem Benutzer (2) und der Verarbeitungseinheit (6) angeordneten Eingangsmittel (98) durch eine Maus (18) verkörpert sind.

19. Expertensystem nach den Patentansprüchen 1-11, **dadurch gekennzeichnet, dass** die zwischen dem Benutzer (2) und der Verarbeitungseinheit (6) angeordneten Eingangsmittel (98) durch ein Touchscreen (19) verkörpert sind.

20. Expertensystem nach den Patentansprüchen 1-11, **dadurch gekennzeichnet, dass** die zwischen dem Benutzer (2) und der Verarbeitungseinheit (6) angeordneten Eingangsmittel (98) durch einen magnetischen Leser (20) verkörpert sind.

21. Expertensystem nach den Patentansprüchen 1-11, **dadurch gekennzeichnet, dass** die zwischen dem Benutzer (2) und der Verarbeitungseinheit (6) angeordneten Eingangsmittel (98) durch einen optischen Leser (21) verkörpert sind.

22. Expertensystem nach den Patentansprüchen 1-11, **dadurch gekennzeichnet, dass** die zwischen dem Benutzer (2) und der Verarbeitungseinheit (6) angeordneten Eingangsmittel (98) durch eine Videokamera (22) verkörpert sind.

23. Expertensystem nach den Patentansprüchen 1-11, **dadurch gekennzeichnet, dass** die zwischen dem Benutzer (2) und der Verarbeitungseinheit (6) angeordneten Eingangsmittel (98) durch einen Sprachsynthetisator (23) verkörpert sind.

24. Expertensystem nach den Patentansprüchen 1-11, **dadurch gekennzeichnet, dass** die zwischen dem Benutzer (2) und der Verarbeitungseinheit (6) angeordneten Eingangsmittel (98) durch einen CD-ROM-Leser (24) verkörpert sind.

25. Expertensystem nach den Patentansprüchen 1-11, **dadurch gekennzeichnet, dass** die zwischen dem Benutzer (2) und der Verarbeitungseinheit (6) angeordneten Eingangsmittel (98) durch einen DVD-Leser (25) verkörpert sind.

26. Expertensystem nach den Patentansprüchen 1-11, **dadurch gekennzeichnet, dass** die zwischen dem Benutzer (2) und der Verarbeitungseinheit (6) angeordneten Eingangsmittel (98) durch einen Diskettenleser (26) verkörpert sind.

27. Expertensystem nach den Patentansprüchen 1-11, **dadurch gekennzeichnet, dass** die zwischen dem Benutzer (2) und der Verarbeitungseinheit (6) angeordneten Eingangsmittel (98) durch ein Modem (27) verkörpert sind.

28. Expertensystem nach den Patentansprüchen 1-27, **dadurch gekennzeichnet, dass** die zwischen dem Benutzer (2) und der Verarbeitungseinheit (6) angeordneten Ausgangsmittel (96) durch einen Monitor (28) verkörpert sind.

29. Expertensystem nach den Patentansprüchen 1-27, **dadurch gekennzeichnet, dass** die zwischen dem Benutzer (2) und der Verarbeitungseinheit (6) angeordneten Ausgangsmittel (96) durch ein Display (29) verkörpert sind.

30. Expertensystem nach den Patentansprüchen 1-27, **dadurch gekennzeichnet, dass** die zwischen dem Benutzer (2) und der Verarbeitungseinheit (6) angeordneten Ausgangsmittel (96) durch einen Drucker (30) verkörpert sind.

31. Expertensystem nach den Patentansprüchen 1-27, **dadurch gekennzeichnet, dass** die zwischen dem Benutzer (2) und der Verarbeitungseinheit (6) angeordneten Ausgangsmittel (96) durch einen Plotter (31) verkörpert sind.

32. Expertensystem nach den Patentansprüchen 1-27, **dadurch gekennzeichnet, dass** die zwischen dem Benutzer (2) und der Verarbeitungseinheit (6) angeordneten Ausgangsmittel (96) durch einen Hörsynthetisator (32) verkörpert sind.

33. Expertensystem nach den Patentansprüchen 1-27, **dadurch gekennzeichnet, dass** die zwischen dem Benutzer (2) und der Verarbeitungseinheit (6) angeordneten Ausgangsmittel (96) durch einen CD-Brenner (33) verkörpert sind.

34. Expertensystem nach den Patentansprüchen 1-27, **dadurch gekennzeichnet, dass** die zwischen dem Benutzer (2) und der Verarbeitungseinheit (6) angeordneten Ausgangsmittel (96) durch ein Modem (27) verkörpert sind.

35. Expertensystem nach einem der vorstehenden Patentansprüche, **dadurch gekennzeichnet, dass** die Mittel zum Austausch von Informationen (99) zwischen der Speichervorrichtung (4) und dem Terminal (5) eine physische Verbindung über Kabel enthalten.

36. Expertensystem nach den Patentansprüchen 1-34, **dadurch gekennzeichnet, dass** die Mittel zum Austausch von Informationen (99) zwischen der Speichervorrichtung (4) und dem Terminal (5) eine kabellose Verbindung enthalten.

37. Expertensystem nach den Patentansprüchen von 35 bis 36, **dadurch gekennzeichnet, dass** die Speichervorrichtung (4) ein Server ist.

38. Expertensystem nach Patentanspruch 37, **dadurch gekennzeichnet, dass** die Speichervorrichtung (4) ein Intranet-Server ist.

39. Expertensystem nach einem der Patentansprüche von 35 bis 38, **dadurch gekennzeichnet, dass** die Speichervorrichtung (4) ein Internet-Server ist.

40. Expertensystem nach einem der Patentansprüche von 35 bis 38, **dadurch gekennzeichnet, dass** die Speichervorrichtung (4) eine Personal-Vorrichtung ist.

41. Expertensystem nach Patentanspruch 40, **dadurch gekennzeichnet, dass** die Speichervorrichtung (4) eine Chipkarte ist.

42. Expertensystem nach einem der vorstehenden Patentansprüche, **dadurch gekennzeichnet, dass** das Kommunikationsterminal (5) mit einer Schnittstelle (35) versehen ist, um mit wenigstens einer dem Benutzer (2) zugedachten Übungsstation (34) zusammenzuwirken.

43. Expertensystem nach Patentanspruch 42, **dadurch gekennzeichnet, dass** die Übungsstation (34) eine Herz-Kreislauf-Trainingsmaschine enthält.

44. Expertensystem nach Patentanspruch 42, **dadurch gekennzeichnet, dass** die Schnittstelle (35) dazu bestimmt ist, wenigstens die Steuerfunktionen der Herz-Kreislauf-Trainingsmaschine zu verwalten.

45. Expertensystem nach Patentanspruch 43 und 44, **dadurch gekennzeichnet, dass** die Schnittstelle (35) dazu bestimmt ist, die an der Herz-Kreislauf-Trainingsmaschine wiederholbaren Trainingsweisen zu verwalten.

46. Expertensystem nach Patentanspruch 42, **dadurch gekennzeichnet, dass** die Übungsstation (34) eine Gegengewichtmaschine enthält, wobei das Terminal (5) in der Lage ist, den Benutzer (2) mit Anweisungen zum Durchführen eines Trainingsprogramms an der genannten Gegengewichtmaschine zu versorgen.

47. Expertensystem nach wenigstens einem der Patentansprüche von 42 bis 46, **dadurch gekennzeichnet, dass** die genannte Schnittstelle (35) aus einer RS 232-Karte besteht.

48. Expertensystem nach wenigstens einem der Patentansprüche von 42 bis 46, **dadurch gekennzeichnet, dass** die genannte Schnittstelle (35) aus einer USB-Karte besteht.

49. Expertensystem nach einem beliebigen der vorstehenden Patentansprüche, **dadurch gekennzeichnet, dass** es ein Kommunikationsprotokoll (UMTS) verwendet, in der Lage, Videoprogramme zu übertragen.

50. Expertensystem nach einem beliebigen der vorstehenden Patentansprüche, **dadurch gekennzeichnet, dass** es ein Kommunikationsprotokoll (WAP) verwendet, in der Lage, Signale drahtlos zu übertragen.

## Revendications

1. Un système expert destiné à un échange interactif d'informations, comprenant un système d'information (3) dédié au traitement d'informations relatives à l'obtention et au maintien de l'efficience de la personne ; un dispositif de mémorisation (4) dans lequel ledit système d'information (3) est mis en oeuvre ; un terminal de communication (5) portable entre un utilisateur (2) et ledit système d'information (3), tel terminal de communication (5) étant équipé d'au moins une unité (6) pour le traitement des informations ainsi que de moyens d'entrée (98) et de moyens de sortie (96) reliés à ladite unité de traitement (6) et destinés à interagir entre cette même unité et ledit utilisateur (2) ; des moyens d'échange des informations (99) placés entre ladite unité de traitement (6) et ledit dispositif de mémorisation (4) pour échanger des informations à distance ; des moyens capteurs (97) destinés à mesurer au moins un paramètre d'état de l'utilisateur (2) et à le transmettre à ladite unité de traitement (6) et au système d'information (3) ; ledit système expert étant **caractérisé en ce que** lesdits moyens capteurs (97) mesure au moins un paramètre d'état mécanique de l'utilisateur (2) et le transmet à l'unité de traitement (6) et au système d'information (3) de manière à exécuter un calcul pour déterminer la dépense d'énergie de l'utilisateur (2) et permettre la préparation de programmes d'activité scientifiquement ciblés pour l'utilisateur (2) en question ; et **en ce qu'**il comprend des moyens de rétroaction (95) destinés à guider l'exécution de l'activité de l'utilisateur (2) sur la base de ces programmes d'activité scientifiquement ciblés.

2. Le système expert selon la revendication 1, **caractérisé en ce que** lesdits moyens capteurs (97) sont conçus pour mesurer un paramètre de position instantanée de l'utilisateur (2).

3. Le système expert selon la revendication 1, **caractérisé en ce que** lesdits moyens capteurs (97) comprennent un indicateur de position absolue de type satellite (8).

4. Le système expert selon une des revendications précédentes 1 et 3-4, **caractérisé en ce que** ledit terminal de communication (5) est fixe.

5. Le système expert selon la revendication 4, **caractérisé en ce que** ledit terminal (5) comprend un ordinateur personnel (9).

6. Le système expert selon la revendication 1, **caractérisé en ce que** ledit terminal (5) est un ordinateur de poche (10).

7. Le système expert selon la revendication 1, **caractérisé en ce que** ledit terminal portable (5) est un téléphone portable (11).

8. Le système expert selon la revendication 7, **caractérisé en ce que** ledit téléphone portable (11) est du type cellulaire.

9. Le système expert selon une des revendications de 1 à 3 et de 6 à 8, **caractérisé en ce que** ledit terminal (5) peut être porté par l'utilisateur (2).

10. Le système expert selon la revendication 9, **caractérisé en ce que** ledit terminal (5) est associé à une sangle.

11. Le système expert selon la revendication 9, **caractérisé en ce que** ledit terminal (5) peut être porté sur le poignet de l'utilisateur (2).

12. Le système expert selon une des revendications précédentes, **caractérisé en ce que** lesdits moyens d'entrée (98) placés entre ledit utilisateur (2) et ladite unité de traitement (6) sont constitués par un clavier (12).

13. Le système expert selon les revendications 1-11, **caractérisé en ce que** lesdits moyens d'entrée (98) placés entre ledit utilisateur (2) et ladite unité de traitement (6) sont constitués par une tablette graphique (13).

14. Le système expert selon les revendications 1-11, **caractérisé en ce que** lesdits moyens d'entrée (98) placés entre ledit utilisateur (2) et ladite unité de traitement (6) sont constitués par un scanner (14).

15. Le système expert selon les revendications 1-11, **caractérisé en ce que** lesdits moyens d'entrée (98) placés entre ledit utilisateur (2) et ladite unité de traitement (6) sont constitués par un stylo optique (15).

16. Le système expert selon les revendications 1-11, **caractérisé en ce que** lesdits moyens d'entrée (98) placés entre ledit utilisateur (2) et ladite unité de traitement (6) sont constitués par un joystick (16).

17. Le système expert selon les revendications 1-11, **caractérisé en ce que** lesdits moyens d'entrée (98) placés entre ledit utilisateur (2) et ladite unité de traitement (6) sont constitués par une trackball (17).

18. Le système expert selon les revendications 1-11, **caractérisé en ce que** lesdits moyens d'entrée (98) placés entre ledit utilisateur (2) et ladite unité de traitement (6) sont constitués par une souris (18).

19. Le système expert selon les revendications 1-11, **caractérisé en ce que** lesdits moyens d'entrée (98) placés entre ledit utilisateur (2) et ladite unité de traitement (6) sont constitués par un écran tactile (19).

20. Le système expert selon les revendications 1-11, **caractérisé en ce que** lesdits moyens d'entrée (98) placés entre ledit utilisateur (2) et ladite unité de traitement (6) sont constitués par un lecteur magnétique (20).

21. Le système expert selon les revendications 1-11, **caractérisé en ce que** lesdits moyens d'entrée (98) placés entre ledit utilisateur (2) et ladite unité de traitement (6) sont constitués par un lecteur optique (21).

22. Le système expert selon les revendications 1-11, **caractérisé en ce que** lesdits moyens d'entrée (98) placés entre ledit utilisateur (2) et ladite unité de traitement (6) sont constitués par une caméra vidéo (22).

23. Le système expert selon les revendications 1-11, **caractérisé en ce que** lesdits moyens d'entrée (98) placés entre ledit utilisateur (2) et ladite unité de traitement (6) sont constitués par un synthétiseur vocal (23).

24. Le système expert selon les revendications 1-11, **caractérisé en ce que** lesdits moyens d'entrée (98) placés entre ledit utilisateur (2) et ladite unité de traitement (6) sont constitués par un lecteur de CD-ROM (24).

25. Le système expert selon les revendications 1-11, **caractérisé en ce que** lesdits moyens d'entrée (98) placés entre ledit utilisateur (2) et ladite unité de traitement (6) sont constitués par un lecteur de DVD (25).

26. Le système expert selon les revendications 1-11, **caractérisé en ce que** lesdits moyens d'entrée (98) placés entre ledit utilisateur (2) et ladite unité de traitement (6) sont constitués par un lecteur de disquette (26).

27. Le système expert selon les revendications 1-11, **caractérisé en ce que** lesdits moyens d'entrée (98) placés entre ledit utilisateur (2) et ladite unité de traitement (6) sont constitués par un modem (27).

28. Le système expert selon les revendications 1-27, **caractérisé en ce que** lesdits moyens de sortie (96) placés entre ledit utilisateur (2) et ladite unité de traitement (6) sont constitués par un moniteur (28).

29. Le système expert selon les revendications 1-27, **caractérisé en ce que** lesdits moyens de sortie (96) placés entre ledit utilisateur (2) et ladite unité de traitement (6) sont constitués par un afficheur (29).

30. Le système expert selon les revendications 1-27, **caractérisé en ce que** lesdits moyens de sortie (96) placés entre ledit utilisateur (2) et ladite unité de traitement (6) sont constitués par une imprimante (30).

31. Le système expert selon les revendications 1-27, **caractérisé en ce que** lesdits moyens de sortie (96) placés entre ledit utilisateur (2) et ladite unité de traitement (6) sont constitués par un traceur (31).

32. Le système expert selon les revendications 1-27, **caractérisé en ce que** lesdits moyens de sortie (96) placés entre ledit utilisateur (2) et ladite unité de traitement (6) sont constitués par un synthétiseur audio (32).

33. Le système expert selon les revendications 1-27, **caractérisé en ce que** lesdits moyens de sortie (96) placés entre ledit utilisateur (2) et ladite unité de traitement (6) sont constitués par un mastérisateur (33).

34. Le système expert selon les revendications 1-27, **caractérisé en ce que** lesdits moyens de sortie (96) placés entre ledit utilisateur (2) et ladite unité de traitement (6) sont constitués par un modem (27).

35. Le système expert selon une des revendications précédentes, **caractérisé en ce que** lesdits moyens (99) d'échange des informations, entre ledit dispositif de mémorisation (4) et ledit terminal (5), comprennent une connexion physique via câble.

36. Le système expert selon les revendications 1-34, **caractérisé en ce que** lesdits moyens (99) d'échange des informations entre ledit dispositif de mémorisation (4) et ledit terminal (5) comprennent une connexion obtenue en mode sans fil.

37. Le système expert selon les revendications de 35 à 36, **caractérisé en ce que** ledit dispositif de mémorisation (4) est un serveur.

38. Le système expert selon la revendication 37, **caractérisé en ce que** ledit dispositif de mémorisation (4) est un serveur intranet.

39. Le système expert selon une des revendications de 35 à 38, **caractérisé en ce que** ledit dispositif de mémorisation (4) est un serveur Internet.

40. Le système expert selon une des revendications de 35 à 38, **caractérisé en ce que** ledit dispositif de mémorisation (4) est un ordinateur personnel.

41. Le système expert selon la revendication 40, **caractérisé en ce que** ledit dispositif de mémorisation (4) est une carte intelligente.

42. Le système expert selon une des revendications précédentes, **caractérisé en ce que** ledit terminal de communication (5) est équipé d'une interface (35) pour interagir avec au moins une station d'exercice (34) dédiée à l'utilisateur (2).

43. Le système expert selon la revendication 42, **caractérisé en ce que** ladite station d'exercice (34) comprend une machine pour l'entraînement cardiovasculaire.

44. Le système expert selon la revendication 42, **caractérisé en ce que** ladite interface (35) est conçue pour gérer au moins les fonctionnalités de contrôle de la machine pour l'entraînement cardiovasculaire.

45. Le système expert selon les revendications 43 et 44, **caractérisé en ce que** ladite interface (35) est conçue pour gérer les modes d'entraînement pouvant être reproduits sur la machine pour l'entraînement cardiovasculaire.

46. Le système expert selon la revendication 42, **caractérisé en ce que** ladite station d'exercice (34) comprend une machine à contrepoids, ledit terminal (5) étant destiné à fournir à l'utilisateur (2) les instructions nécessaires pour l'exécution d'un programme d'entraînement sur ladite machine à contrepoids.

47. Le système expert selon au moins une des revendications de 42 à 46, **caractérisé en ce que** ladite interface (35) est mise en oeuvre par une carte RS 232.

48. Le système expert selon au moins une des revendications de 42 à 46, **caractérisé en ce que** ladite interface (35) est mise en oeuvre par une carte USB.

49. Le système expert selon l'une quelconque des revendications précédentes, **caractérisé en ce qu'**il peut utiliser un protocole de communication (UMTS) à même de transmettre des programmes vidéo.

50. Le système expert selon une des revendications précédentes, **caractérisé en ce qu'**il peut utiliser un protocole de communication (WAP) à même de transmettre des signaux sans fil.
